(19) Europäisches Patentamt
European Patent Office
Office européen des brevets

(11) Numéro de publication: **0 032 889**
**B1**

(12) # FASCICULE DE BREVET EUROPEEN

(45) Date de publication du fascicule du brevet: **15.02.84**

(21) Numéro de dépôt: **81870002.3**

(22) Date de dépôt: **09.01.81**

(51) Int. Cl.³: **C 07 D 491/22,** A 61 K 31/475 // (C07D491/22, 311/00, 221/00, 221/00, 209/00)

(54) **Nouveaux dérivés du type diméthyl-1,10 oxayohimbane et médicaments les contenant.**

(30) Priorité: **09.01.80 BE 198889**

(43) Date de publication de la demande: **29.07.81 Bulletin 81/30**

(45) Mention de la délivrance du brevet: **15.02.84 Bulletin 84/7**

(84) Etats contractants désignés: **BE CH DE FR GB IT LI NL SE**

(56) Documents cités:
**FR - A - 2 222 374**
**GB - A - 1 378 076**
**GB - A - 2 018 751**

**Le dossier contient des informations techniques présentées postérieurement au dépôt de la demande et ne figurant pas dans le présent fascicule.**

(73) Titulaire: **OMNICHEM Société anonyme, 12 Avenue de Broqueville, B-1150 Bruxelles (BE)**.

(72) Inventeur: **Hannart, Jean Alfred Alphonse, avenue d'El Pirere 25, B-1302 Dion Valmont (BE)**

**EP 0 032 889 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Nouveaux dérivés du type diméthyl-1,10 oxayohimbane et médicaments les contenant

La présente invention concerne de nouveaux dérivés pentacycliques du type oxayohimbane méthylés en position 1 et 10 et les compositions pharmaceutiques les contenant.

Les composés de l'invention répondent à la formule générale suivante:

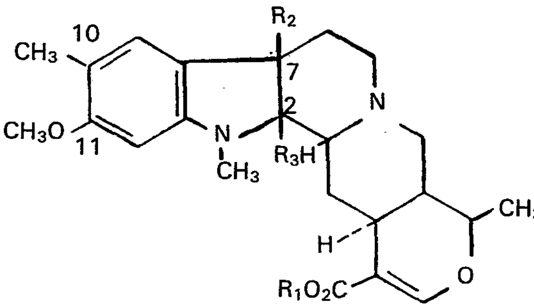

(I)

dans laquelle R représente un groupe aminoalkyle comportant de 1 à 8 atomes de carbone, ou un atome d'hydrogène;
$R_2$et $R_3$ représentent soit des atomes d'hydrogène, soit ensemble, une liaison carbone-carbone supplémentaire, ainsi que ses sels d'addition d'acide minéral ou organique.

Plus particulièrement, parmi les dérivés de l'invention sont sompris des n-$\alpha$-méthyl dihydro-2,7 oxayohimbanes disubstitués, en position 10 et 11, sur le noyau aromatique.

Les composés de départ utilisés dans le procédé de la présente invention sont les dérivés indoliques pentacycliques 11-méthoxylés correspondants de la formule II

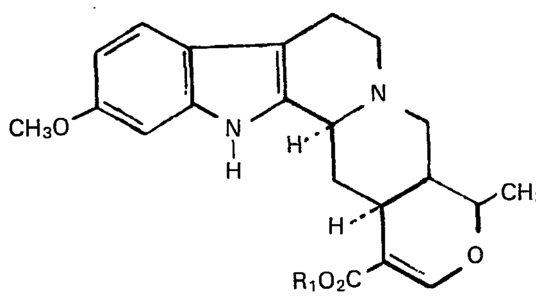

(II)

Les composés de l'invention sont plus particulièrement obtenus avantageusement à partir de la tétraphylline de formule II dans laquelle $R_1 = CH_3$.

Il a en effet été démontré que la tétraphylline peut être facilement transformée en les esters de formule II dans laquelle $R_1$ représente un groupe tel que le groupe diméthyl aminoéthyle, morpholinoéthyle, N-méthylpipérazinoéthyle ou pipéridinoéthyle (brevets belges 794 950 et 780 854).

La tétraphylline elle-même est un alcaloïde facilement isolé en grande quantité à partir de racines de Rauwolfia (Djerassi, Fishman, Chem. Ind. 627, 1955).

A partir des composés de l'invention de formule I dans laquelle $R_1$ représente un groupe méthyle, il est également possible d'obtenir, de manière classique, les acides tétraphylliques correspondants qui peuvent être estérifiés pour fournir des composés dans lesquels $R_1$ représente un aminoalkyle comportant de 1 à 8 atomes de carbone (voir brevets belges cités ci-avant).

De nombreux dérivés possédant le squelette oxayohimbane dont la tétraphylline et l'ajmalicine sont dérivés, se sont révélés des composés aux propriétés hypotensives vasodilatatrices et débitmétriques intéressantes.

Le brevet français n° 7 216 822 de la Demanderesse décrit ainsi la synthèse et les propriétés pharmacologiques de dérivés aminoéthyliques de la tétraphylline.

Les composés de l'invention se sont également révélés actifs dans de nombreux tests pharmacologiques et sont donc susceptibles d'être utilisés comme principe actif de médicaments. Les dérivés de l'invention peuvent être également utilisés comme produits intermédiaires pour la synthèse d'autres substances à application thérapeutique.

En particulier, les composés dihydro-2,7 tétraphyllinate de l'invention se sont révélés actifs comme antiarythmiques dans le test à l'aconitine.

Ce test à été réalisé sur des rats anesthésiés maintenus en respiration artificielle et pour lesquels on enregistre en continu l'électrocardiogramme en trois dérivations DI, DII, DIII.

Pour les rats témoins, la perfusion d'une solution d'aconitine engendre, après deux à trois minutes, l'apparition d'extrasystoles ventriculaires (E.S.V.). Les animaux traités auparavant avec des drogues antiarythmiques allongent la latence d'apparition de ces troubles cardiaques.

Les composés I sous forme base sont injectés par voie i.v. en solution de HCl $\pm$ 0.02 N, cinq minutes avant le début de la perfusion d'aconitine.

Le tableau suivant reprend les résultats obtenus avec quatre produits de référence (lidocaine, quinidine, ajmaline et aprindine) comparés aux résultats obtenus avec quatre composés de la présente invention:

- (Ia): diméthyl-1,10 dihydro-2,7 tétraphyllinate de diméthylamino-2-éthyle
- (Ib): diméthyl-1,10 dihydro-2,7 tétraphyllinate de N-méthylpipérazino-2-éthyle
- (Ic): diméthyl-1,10 dihydro-2,7 tétraphyllinate de pipéridino-2-éthyle
- (Id): diméthyl-1,10 dihydro-2,7 tétraphyllinate de diéthylamino-2-éthyle.

TABLEAU

| Produits de référence | Dose I.V. mole/kg | Temps d'apparition de la première E.S.V. (en minutes décimales) $\overline{X}$ | s.e.m.* | Réponse en % du temps observé des témoins |
|---|---|---|---|---|
| LIDOCAINE | 36,93 | 3,12 | 0,17 | 119 |
| | 55,39 | 3,90 | 0,30 | 147 |
| | 73,86 | 4,21 | 0,27 | 186 |
| QUINIDINE | 13,39 | 3,38 | 0,29 | 127 |
| | 26,78 | 3,38 | 0,32 | 176 |
| | 40,17 | 4,48 | 0,33 | 198 |
| AJMALINE | 3,063 | 3,31 | 0,16 | 146 |
| | 6,126 | 3,94 | 0,27 | 168 |
| | 12,2512 | 5,32 | 0,31 | 235 |
| APRINDINE | 3,48 | 3,19 | 0,25 | 126 |
| | 6,97 | 5,70 | 0,45 | 225 |
| | 13,93 | 8,88 | 1,22 | 351 |
| ------------------ | | | | |
| Ia | 2,66 | 3,80 | 0,33 | 152 |
| | 4,26 | 4,90 | 0,81 | 183 |
| | 5,32 | 5,16 | 0,68 | 204 |
| Ib | 1,525 | 4,25 | 0,23 | 158 |
| | 3,049 | 5,24 | 0,58 | 207 |
| Ic | 1,32 | 3,49 | 0,35 | 138 |
| | 1,98 | 4,10 | 0,31 | 169 |
| | 2,65 | 4,43 | 0,33 | 186 |
| Id | 0,88 | 4,23 | 0,67 | 154 |
| | 1,77 | 5,33 | 0,56 | 194 |
| | 2,66 | 9,19 | 1,85 | 334 |

* s.e.m.: somme des écarts à la moyenne.

Les composés de l'invention, et en particulier le composé Id, se sont révélés être d'activité égale ou supérieure aux composés de référence bien connus pour leur bonne activité antiarythmique.

La $DL_{50}$ i.g. pour le composé Id est de 406 mg/kg chez la souris, cette dose étant déterminée graphiquement selon la méthode de Lichtfield et Wilcoxon (J. Pharmacol. Exp. Thér., 1946, 96, 99).

Les exemples suivants illustrent le procédé de préparation des composés de l'invention, procédé qui n'est pas objet de ladite invention.

*Exemple 1*

*Diméthyl-1,10 dihydro-2,7 tétraphylline*

A une solution de 2 g de tétraphylline dans 50 ml d'acide trifluoroacétique, on ajoute par petites portions 1,4 g de $NaBH_3CN$ ou de $NaBH_4$.

Après refroidissement au bain de glace, on ajoute 20 ml de formaldéhyde aqueux à 37%. La vitesse d'introduction de l'aldéhyde est réglée de telle sorte que la température du milieu reste inférieure à + 10°C. La réaction se poursuit par l'addition de 1 g de $NaBH_3CN$ ou de $NaBH_4$. Aorès dix minutes d'agitation, le mélange est noyé à l'eau glacée, alcalinisé à l'ammoniaque dilué et extrait trois fois par 200 ml de $CH_2Cl_2$. La phase organique séchée, filtrée et évaporée à sec fournit un résidu (2,63 g) qui cristallise dans le méthanol.

On obtient ainsi 1,89 g (87%) du composé dérivé pur.

Fusion: 200,5°C

$\alpha_D$: + 86,6 ($CHCl_3$; c=0,25)

U.V. (méthanol) max: 213 (4,48); 241 (4,24); 303 (3,73) — min: 229 et 276.

I.R. (KBr): 1620 et 1705 $cm^{-1}$.

Spectre de masse: 412 ($M^+$), 397, 381, 262, 242, 238, 237, 236, 226, 225, 224, 222, 210, 209, 202, 188, 168, 154, 150, 149, 139, 132, 122.

R.M.N. du $^1H$: signaux à ppm: 7,56 s(1H)$C_{(17)}$H; 6,83 s(1H)$C_{(12)}$H; 6,24 s(1H)$C_{(9)}$H; 3,78 s(3H)-$C_{(11)}OCH_3$; 3,70 s(3H) $COOCH_3$; 2,86 s(3H)$N_{(1)}$-$CH_3$; 2,16 s(3H)$C_{(10)}$-$CH_3$; 1,12 d(3H)$C_{(18)}$-H (J=7Hz).

*Exemple 2*

*Acide diméthyle-1,10 dihydro-2,7 tétraphyllique*

15 g de diméthyl-1,10 dihydro-2,7 tétraphylline en suspension dans un mélange de 150 ml de méthanol et 150 ml de NaOH à 5% sont portés à reflux sous atmosphère d'argon. Après 2h30, la solubilisation est totale et la saponification complète.

Le milieu refroidit et l'alcool est chassé par distillation sous vide.

Le pH est alors abaissé jusqu'aux environs de 6,5 par l'addition d'acide acétique à 50% dans l'eau. Un abondant précipité blanc est filtré, rincé à l'eau distillée et séché sous vide à 80°C pendant deux heures.

Les 13,9 g (soit un rendement de 96%) d'acide brut ainsi obtenus sont recristallisés dans un mélange méthanol - chlorure de méthylène.

Fusion: 284-288°C ($CH_2Cl_2$-$CH_3OH$)

U.V. (méthanol; c=10,196 mg/l) max: 209 (4,60); 301 (3,69) — inflexion: 240 (4,17) — min: 275 (3,19)

I.R. (KBr): 3420, 2460, 1670 et 1600 $cm^{-1}$.

*Exemple 3*

*Diméthyl-1,10 dihydro-2,7 tétraphyllinate de diméthylamino-2 éthyle (Ia)*

A une solution de 13,78 g de tétraphyllinate de diméthylamino éthyle dans 345 ml d'acide trifluoroacétique, on additionne par petites portions 9,7 g de $NaBH_3CN$ sur une période de 45 minutes.

Après refroidissement au bain de glace, on ajoute goutte à goutte 138 ml de formaldéhyde à 37% puis 6,9 g de $NaBH_3CN$. Une agitation très vigoureuse et un courant d'argon sont nécessaires pour empêcher le $NaBH_3CN$ de s'enflammer.

Le mélange est versé sur de la glace, alcalinisé à l'ammoniaque puis épuisé au chlorure de méthylène. Les extraits sont lavés à l'eau, séchés et évaporés à sec sous vide, fournissant un résidu (11,67 g soit 79%) qui est recristallisé dans le méthanol ou l'éther.

Fusion: 187-188°C (méthanol)

$\alpha_D$: + 80°C (c=0,25; $CHCl_3$)

U.V. (méthanol) c=14,95 mg/l: max: 212 (4,50); 242 (4,32); 301 (3,85) — min: 228 (4,25); 275 (3,38)

I.R. (KBr) bandes à 1695 et 1610 $cm^{-1}$

R.M.N.: 7,62 s(1H)$C_{(17)}H$; 6,87 s(1H)$C_{(12)}H$; 6,27 s(1H)$C_{(9)}H$; 4,27 J=6cps(2H) $CO_2CH_2$; 3,83 s (3H)$C_{(11)}$-$OCH_3$; 2,90 s(3H) N-$CH_3$; 2,32 s(6H)-$N(CH_3)_2$; 2,13 s(3H)$C_{(10)}CH_3$; 1,13 d J=7cps (3H)$C_{(18)}H_3$.
Spectre de masse: $M^+$ à 469.

*Exemple 4*

*Diméthyl-1,10 dihydro-2,7 tétraphyllinate de diéthylamino-2 éthyle (Id)*

A une solution de 18,4 g de tétraphyllinate de diéthylaminoéthyle dans 460 ml d'acide trifluoroacétique, on ajoute sur une période de une heure 13,1 g de $NaBH_3CN$. Quinze minutes après la fin de l'addition du réactif, on refroidit le mélange à zéro degré et on ajoute 184 ml de solution aqueuse à 37% de formaldehyde.

Après une agitation de 15 minutes à température ambiante, on ajoute par petites portions 9,2 g de $NaBH_3CN$.

Le mélange est versé sur de la glace, alcalinisé à l'ammoniaque et épuisé au $CH_2Cl_2$.

Les phases organiques lavées à l'eau, séchées et évaporées à sec abandonnent un résidu qui pèse 17,23 g dont la cristallisation dans l'ecétone fournit 9,1 g de cristaux.

Fusion: 121-122°C (acétone)
$\alpha_D$: + 88° (c=0,25; $CHCl_3$)
U.V. (méthanol) c=15,2 mg/l: max: 243 (4,26); 302 (3,71) — min: 228 (4,15); 275 (2,23)
I.R. (KBr( bandes à 1690, 1610, 818, 765 (aromatique 1,2, 4,5 tétrasubstitué)
R.M.N. ($CDCl_3$): 7,60 (s,1H,$H_{17}$); 6,88 (s,1H,$H_{12}$); 6,45 (s,1H,$H_9$); 4,25 (t,2H,$CO_2CH_2$); 3,85 (s,3H, $OCH_3$); 2,93 (s,3H,N-$CH_3$); 2,17 (s.3H,Ar-$CH_3$); 1,17 (d,$7H_2$,3H,$C_{18}H_3$); 1,07 (t, 6H, $CH_2$-$\underline{CH_3}$)
Spectre de Masse: $M^+$ à 497,7; 381, 309, 294, 188, 160, 100, 86.

*Exemple 5*

*Diméthyl-1,10 dihydro-2,7 tétraphyllinate de morpholino-2 éthyle*

A une solution de 11 g de tétraphyllinate d'éthyle morpholine dans 275 ml d'acide trifluoroacétique, on additionne par petites portions 7,7 g de $NaBH_3CN$ sur une période de trente minutes. Après refroidissement au bain de glace, on ajoute goutte à goutte 110 ml de formaldéhyde à 37% puis 5,5 g de $NaBH_3CN$.

Une agitation très vigoureuse et un courant d'argon sont nécessaires pour empêcher le $NaBH_3CN$ de s'enflammer.

Le mélange est versé sur de la glace, alcalinisé à l'ammoniaque puis épuisé au chlorure de méthylène.

Les extraits lavés à l'eau, séchés sur $MgSO_4$ et évaporés à sec sous vide fournissent un résidu (8,96 g soit rendement: 80%) qui est recristallisé dans le méthanol.

Fusion: 169-170°C (méthanol)
$\alpha_D$: + 75° (c=0,25; $CHCl_3$)
U.V. (méthanol) c=19,96 mg/l: max: 211 (4,54); 242 (4,29); 301 (3,78) — min: 228 (4,21); 274 (3,15)
I.R. (KBr): bandes à 1615 et 1705 $cm^{-1}$
R.M.N.: 7,60 s(1H)$C_{(17)}H$; 6,87 s(1H)$C_{(12)}H$; 6,30 s(1H)$C_{(9)}H$; 4,33 t J=6cps (2H) $CO_2CH_2$-; 3,84 s(3H)$C_{(11)}$-$OCH_3$; 2,90 s(3H)N-$CH_3$; 2,16 s(3H)-$C_{(10)}CH_3$; 1,16 d J=7cps (3H)$C_{(18)}H_3$.
Spectre de masse: $M^+$ à 511.

*Exemple 6*

*Diméthyl-1,10 dihydro-2,7 tétraphyllinate de pyrrolidino-2 éthyle*

A une solution de 14,5 g de tétraphyllinate d'éthyle pyrrolidine dans 360 ml d'acide trifluoroacétique, on additionne par petites portions 10,23 g de $NaBH_3CN$ sur une période de 45 minutes. Après refroidissement au bain de glace, on ajoute goutte à goutte 145 ml de formaldéhyde à 37% puis 7,27 g de $NaBH_3CN$ sous agitation vigoureuse et sous argon.

Le mélange est versé sur de la glace, alcalinisé à l'ammoniaque puis épuisé au chlorure de méthylène.

Les extraits lavés à l'eau, séchés sur $MgSO_4$ et évaporés à sec sous vide fournissent un résidu (10,5 g; rendement: 68%) qui est recristallisé dans l'acétone.

Fusion: 144-145°C (acétone)
$\alpha_D$: + 78° (c=0,25; $CHCl_3$)
U.V. (méthanol, c=15,64 mg/l) max: 212 (4,54); 241 (4,31); 30 (3,77) — min: 228 (4,26); 275 (3,24).
I.R. (KBr) bandes à 1620 et 1710 $cm^{-1}$.
R.M.N.: 7,63 s(1H)$C_{(17)}H$; 6,88 s(1H)$C_{(12)}H$; 6,28 s(1H)$C_{(9)}H$; 4,33 t J=6cps (2H) $CO_2$-$CH_2$; 3,83 s(3H)$C_{(11)}OCH_3$; 2,88 s(3H) N-$CH_3$; 2,13 s(3H)-$C_{(10)}H_3$; 1,13 d J=7cps (3H)$C_{(18)}H_3$.
Spectre de masse: $M^+$ à 495.

*Exemple 7*

*Diméthyl-1,10 dihydro-2,7 tétraphyllinate de n-méthyle pipérazino-2 éthyle (Ib)*

A une solution de 13,64 g de tétraphyllinate de N-méthyle pipérazinoéthyle dans 340 ml d'acide trifluoroacétique, on additionne par petites portions 9,54 g de $NaBH_3CN$ sur une période de 45 minutes. Après refroidissement au bain de glace, on ajoute goutte à goutte 136 ml de formaldéhyde à 37% puis 6,82 g de $NaBH_3CN$ sous agitation vigoureuse et sous argon.

Le mélange est versé sur de la glace, alcalinisé à l'ammoniaque puis épuisé au chlorure de méthylène.

Les extraits réunis, lavés à l'eau, séchés sur $MgSO_4$ et évaporés à sec sous vide, fournissent un résidu (11,21 g; rendement: 77,5%) qui cristallise dans l'éther.

Fusion: 147-148°C (éther)
$\alpha_D$: + 68° (c=0,25; $CHCl_3$)
U.V. (méthanol; c=14,66 mg/l) max: 212 (4,52); 242 (4,28); 301 (3,80); — min: 228 (4,20); 275 (3,35).
I.R. (KBr) bandes à 1622 et 1698 $cm^{-1}$.
R.M.N.: 7,60 s(1H)$C_{(17)}H$; 6,88 s(1H)$C_{(12)}H$; 6,30 s(1H)$C_{(9)}H$; 4,33 t J=6cps (2H) $CO_2CH_2$-; 3,83 s(3H)$C_{(11)}OCH_3$; 2,90 s(3H)$N_{(1)}CH_3$; 2,57 s(large

8H) de la pipérazine; 2,31 s(3H)N'-$CH_3$; 2,15 s(3H)$C_{(10)}CH_3$; 1,15 d J=7cps(3H)$C_{(18)}H_3$.
Spectre de masse: $M^+$ à 524.

*Exemple 8*

*Diméthyl-1,10 dihydro-2,7 tétraphyllinate de pipéridino-2 éthyle (Ic)*

A une solution de 14,25 g de tétraphyllinate de pipéridinoéthyle dans 356 ml d'acide trifluoroacétique, on additionne par petites portions 9,97 g de $NaBH_3CN$ sur une période de 45 minutes. Après refroidissement au bain de glace, on ajoute goutte à goutte 143 ml de formaldéhyde à 37% puis 7,12 g de $NaBH_3CN$, sous agitation vigoureuse et sous argon.

Le mélange est versé sur de la glace, alcalinisé à l'ammoniaque puis épuisé au chlorure de méthylène.

Les extraits réunis, lavés à l'eau, séchés sur $MgSO_4$ et evaporés à sec sous vide fournissent un résidu (12,25 g soit 81%) qui est cristallisé dans l'éther.

Fusion: 137-138°C (éther)
$\alpha_D$: + 77° (c=0,25; $CHCl_3$)
U.V. (méthanol, c=14,49 mg/l) max: 212 (4,60); 242 (4,42); 301 (3,9) — min: 228 (4,34); 275 (3,47).
I.R. (KBr) bandes à 1705 et 1620 $cm^{-1}$.
R.M.N.: 7,63 s(1H)$C_{(17)}H$; 6,88 s(1H)$C_{(12)}H$; 6,30 s(1H)$C_{(19)}H$; 4,33 t J=6cps(2H) $CO_2CH_2$; 3,87 s(3H)$C_{(11)}OCH_3$; 2,93 s(3H)N-$CH_3$; 2,16 s(3H)-$C_{(10)}CH_3$; 1,13 d J=7cps(3H)$C_{(18)}H_3$.
Spectre de masse: $M^+$ à 509.

*Exemple 9*

*Diméthyl-1,10 tétraphylline (par DDQ)*

A une solution de 7 g de diméthyl-1,10 dihydro-2,7 tétraphylline dans un mélange de 35 ml d'éther et 35 ml de dioxane, on ajoute goutte à goutte à 0°C une solution de 5,80 g (1,5 équivalents) de 2,3 dichloro-5,6 dicyano-1,4 benzoquinone (DDQ) dans 110 ml de dioxane. Après 25 minutes d'agitation, en dehors du bain réfrigérant, on dilue à l'éther et on extrait quatre fois la solution par de l'ammoniaque dilué. L'éther est séché et concentré sous vide. Le résidu (6,41 g soit un rendement de 92%) cristallise dans le méthanol.

Fusion: 194-195°C (méthanol)
$\alpha_D$: — 112° (c=0,25; $CHCl_3$)
U.V. (méthanol, c=15,40 mg/l) max: 231 (4,65); 300 (3,93) — min: 267 (3,76) — plateau: 280-282 (3,82)
I.R. (KBr) bandes à 1605 et 1705 $cm^{-1}$.
R.M.N.: 7,60 s(1H)$C_{(17)}H$; 7,30 s(1H)$C_{(12)}H$; 6,77 s(1H)$C_{(9)}H$; 3,93 s(3H)$C_{(11)}OCH_3$; 3,77 s(6H) $CO_2CH_3$+N-$CH_3$; 2,35 s(3H)$C_{(10)}CH_3$; 1,16 d J=7cps(3H)$C_{(18)}H_3$.
Spectre de masse: $M^+$ à 410.

*Exemple 10*

*Diméthyl-1,10 tétraphylline (par $CuCl_2$)*

A une suspension de 700 mg de $CuCl_2$ dans 2 ml de pyridine, on ajoute une solution de 550 mg de diméthyl-1,10 dihydro-2,7 tétraphylline dans 5 ml de pyridine. On porte au reflux pendant 5 heures sous argon. On dilue à l'eau et on extrait à l'éther.

La phase organique est lavée une fois à l'ammoniaque dilué, séchée et évaporée à sec.

On obtient 0,46 g de résidu soit 84%, qui est cristallisé dans le méthanol.

*Exemple 11*

*Diméthyl-1,10 tétraphylline (oxydation à l'air)*

Une solution méthanolique de chlorhydrate de diméthyl-1,10 dihydro-2,7 tétraphylline (500 mg) est abandonnée pendant 3 jours à l'air libre.

La solution est evaporée à sec. Le résidu repris à l'eau, alcalinisé, extrait au chlorure de méthylène et évaporé, est séparé sur plaques de silice préparatives en éluant du mélange $CH_2Cl_2$-$CH_3OH$ saturé en $NH_3$(99:1). Le produit le moins polaire est l'alcaloïde de départ. Le produit le plus polaire est la diméthyl-1,10 tétraphylline.

*Exemple 12*

*Diméthyl-1,10 tétraphyllinate de pipéridino-2 éthyle*

A 509 mg (1 m.mole) de diméthyl-1,10 dihydro-2,7 tétraphyllinate d'éthyle pipéridine en solution dans un mélange de 7 ml de dioxane et 2 ml d'éther, on ajoute à 0°C une solution de 341 mg (1,5 m.moles) de DDQ dans 3 ml de dioxane sec.

Après une demi heure, on dilue par 200 ml d'éther et on épuise la solution par l'ammoniaque dilué.

L'éther évaporé à sec fournit un résidu (450 mg: rendement: 88%) qui cristalline dans le méthanol.

Fusion: 150-151°C
U.V. (méthanol; c=14,95 mg/l): max: 232 (4,33); 301 (3,58) — min: 269-276 (3,43) — plateau: 280-287 (3,47)
I.R. (KBr) bandes à 1618 et 1690 $cm^{-1}$
Spectre de masse: $M^+$ à 507.

*Exemple 13*

*Diméthyl-1,10 tétraphyllinate de morpholino-2 éthyle*

A 511 mg (1 m.mole) de diméthyl-1,10 dihydro-2,7 tétraphyllinate d'éthyle morpholine en solution dans un mélange de 7 ml de dioxane et 2 ml d'éther, on ajoute à 0°C une solution de 341 mg (1,5 m.moles) de DDQ dans 3 ml de dioxane sec.

Après une demi-heure, la réaction est complète. On dilue par 200 ml d'éther et on épuise la solution par l'ammoniaque dilué.

L'éther évaporé à sec fournit un résidu (400 mg, rendement: 78,6%) qui cristallise dans le méthanol.

Fusion: 168-169°C (méthanol)
U.V. (méthanol; c=16,11 mg/l) max: 234 (4,63); 299 (3,91) — min: 270-272 (3,77) — plateau: 279-286 (3,82)
I.R. (KBr) bandes à 1615 et 1695 $cm^{-1}$
Spectre de masse: $M^+$ à 509.

**Revendications**

1. Oxayohimbanes de formule générale

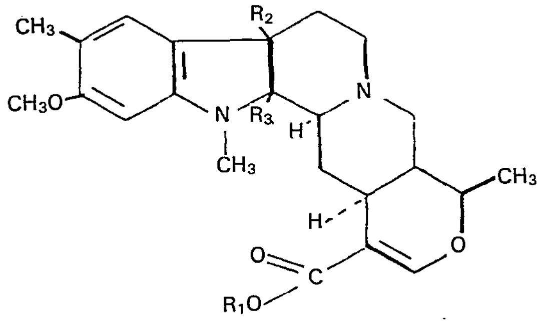

(I)

dans laquelle $R_1$ représente un atome d'hydrogène ou un groupe aminoalkyle comportant de 1 à 8 atomes de carbone et $R_2$ et $R_3$ représentent des atomes d'hydrogène ou, ensemble, une liaison carbone-carbone supplémentaire, ainsi que ses sels d'addition d'acide minéral ou organique.

2. Composé selon la revendication 1, caractérisé en ce qu'il s'agit d'oxayohimbane de formule I dans laquelle $R_2$ et $R_3$ représentent ensemble une liaison supplémentaire.

3. Composé selon la revendication 1, caractérisé en ce qu'il s'agit de dihydro-2,7-oxayohimbanes de formule I dans laquelle $R_2 = R_3 = H$.

4. Composé selon la revendication 1, caractérisé en ce qu'il s'agit du diméthyl-1,10-dihydro-2,7-tétraphyllinate de diéthylamino-2-éthyle.

5. Composition pharmaceutique caractérisée en ce qu'elle contient au moins un des composés selon les revendications 1 à 4, éventuellement sous la forme d'un sel d'addition avec un acide minéral ou organique pharmaceutiquement acceptable.

6. Médicaments suivant la revendication 5 sous la forme de gélules ou de solutés injectables dosés unitairement de 1 à 100 mg de produit actif à prendre en une ou plusieurs doses journalières ou occasionnelles, éventuellement en combinaison avec les adjuvants, diluants, véhicules ou excipients habituels en pharmacie.

7. Compositions pharmaceutiques pour le traitement des troubles du rythme cardiaque caractérisé en ce qu'elles contiennent comme principe actif au moins un des composés définis dans la revendications 1 tel que $R_2 = R_3 = H$.

**Patentansprüche**

1. Oxayohimbane der allgemeinen Formel

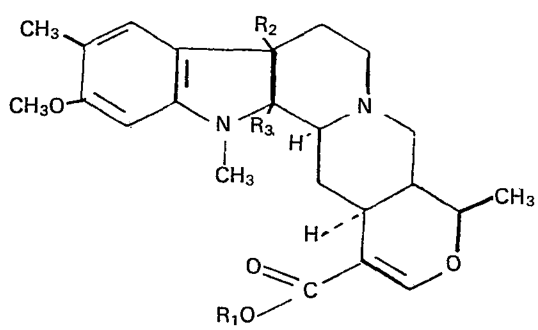

(I)

worin $R_1$ ein Wasserstoffatom oder eine Aminoalkylgruppe mit 1 bis 8 Kohlenstoffatomen bedeutet und $R_2$ und $R_3$ Wasserstoffatome oder gemeinsam eine zusätzliche Kohlenstoff-Kohlenstoffbindung darstellen, sowie ihre Additionssalze mit einer Mineralsäure oder organischen Säure.

2. Verbindungen gemäss Anspruch 1, dadurch gekennzeichnet, dass es sich um Oxayohimban der Formel I handelt, worin $R_2$ und $R_3$ gemeinsam eine zusätzliche Bindung bedeuten.

3. Verbindungen gemäss Anspruch 1, dadurch gekennzeichnet, dass es sich um Dihydro-2,7--oxayohimbane der Formel I handelt, worin $R_2 = R_3 = H$.

4. Verbindungen gemäss Anspruch 1, dadurch gekennzeichnet, dass es sich um Diäthylamino-2--äthyl-dimethyl-1,10-dihydro-2,7-tetraphyllinat handelt.

5. Pharmazeutische Zusammensetzung, dadurch gekennzeichnet, dass sie zumindest eine der Verbindungen gemäss den Ansprüchen 1 bis 4, gegebenenfalls in Form eines Additionssalzes mit einer pharmazeutisch verträglichen Mineral- oder organischen Säure enthält.

6. Arzneimittel gemäss Anspruch 5 in Form von Gelkapseln oder injizierbaren Lösungen mit einer Einheitsdosis von 1 bis 100 mg Wirkstoff für eine Verabreichung in ein oder mehreren täglichen oder gelegentlichen Dosen, gegebenenfalls in Kombination mit in der Pharmazie üblichen Adjuvantien, Verdünnungsmitteln, Trägern oder Excipienten.

7. Pharmazeutische Zusammensetzungen für die Behandlung von Herzrythmusstörungen, dadurch gekennzeichnet, dass sie als Wirkstoff zumindest eine der in Anspruch 1 definierten Verbindungen enthalten, worin $R_2 = R_3 = H$.

**Claims**

1. Oxayohimbanes of the general formula

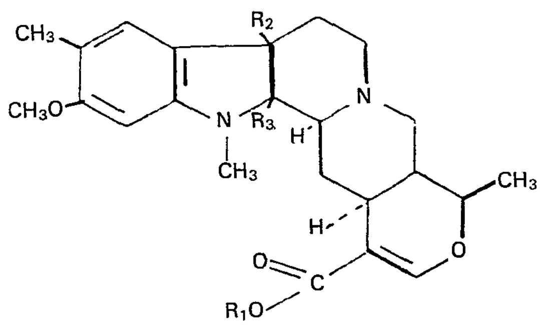

(I)

wherein $R_1$ represents a hydrogen atom or an aminoalkyl group comprising from 1 to 8 carbon atoms and $R_2$ and $R_3$ represent hydrogen atoms or, together, a carbon-carbon bond, and their addition salts with a mineral or organic acid.

2. Compounds according to claim 1, characterised in that they are oxayohimbanes of formula I wherein $R_2$ and $R_3$ represent together an additional bond.

3. Compounds according to claim 1, characterised in that they are dihydro-2,7-oxayohimbanes of formula I wherein $R_2 = R_3 = H$.

4. A compound according to claim 1, characterised in that it is diethylamino-2-ethyl dimethyl-1,10-dihydro-2,7-tetraphyllinate.

5. A pharmaceutical composition characterised in that it comprises at least one of the compounds according to claims 1 to 4, possibly in the form of an addition salt with a pharmaceutically acceptable mineral or organic acid.

6. Medicaments according to claim 5 in the form of capsules or injectable solutes in unit dosage of 1 to 100 mg of active principle to be taken once or several times daily or occasionally, possibly combined with adjuvants, diluents, vehicles or excipients usual in pharmacy.

7. Pharmaceutical compositions for treating cardiac rhythm disorders characterised in that they comprise as active principle at least one of the compounds defined in claim 1 so that $R_2 = R_3 = H$.